Europäisches Patentamt

European Patent Office

Office européen des brevets

(19)

(11) Publication number: **0 220 050 B1**

# EUROPEAN PATENT SPECIFICATION

(12)

(45) Date of publication of patent specification: 06.03.91    (51) Int. Cl.⁵: **A61L 9/18**

(21) Application number: 86307951.3

(22) Date of filing: 14.10.86

(54) Apparatus for purifying air by ultraviolet radiation.

(30) Priority: 14.10.85 BR 8505258
29.10.85 BR 8505540

(43) Date of publication of application:
29.04.87 Bulletin 87/18

(45) Publication of the grant of the patent:
06.03.91 Bulletin 91/10

(84) Designated Contracting States:
AT BE CH DE ES FR GB GR IT LI LU NL SE

(56) References cited:
CH-A- 515 721     DE-A- 2 400 430
FR-A- 1 604 452     FR-A- 2 182 755
FR-A- 2 189 077     US-A- 2 248 618

(73) Proprietor: Keutenedjian, Ubirajara
Rua Austria 95
Sao Paulo(BR)

(72) Inventor: Keutenedjian, Ubirajara
Rua Austria 95
Sao Paulo(BR)

(74) Representative: Diamond, Bryan Clive et al
Gee & Co., Chancery House, Chancery Lane
London WC2A 1QU(GB)

## Description

The present patent application relates to apparatus for the degradation and/or modification of germs and other substances in the atmosphere in order to combat allergies, to benefit health, and which is easily operated by the user.

As is well known, many illnesses, especially those of the respiratory tract, are caused by germs such as bacteria, viruses and fungi and/or substances suspended in the air and which when inhaled by a person having a weakened and/or in any way impaired immunological system, attack the body causing various pathologic effects that may damage the person's body.

In order to combat these illnesses modern medicine offers several resources such as appropriate medicines, inhaling apparatus and others, which although effective can generally only be used when prescribed by a doctor and under his guidance.

A first embodiment of apparatus of the invention for purifying air by causing a stream of air to be subjected to ultraviolet radiation, comprises a vertical housing (1) made of insulating material which is opaque to infra-red, ultra-violet, heat and other radiations, containing infra-red lamps in its lower part (2) and ultra-violet lamps (UVA-UVB-UVC) in its upper part (3), with an inlet aperture (4) provided in the housing wall at the level of the first lamps, through which aperture, when the lamps are illuminated, air to be treated is introduced by convection and above this inlet an outlet for treated air is provided (5) which can be located at an intermediate level of the housing so as to define an air accumulation chamber (6) located above said air outlet, in which chamber air is confined and exposed to the action of the lamps, part of this air being mixed with the treated air leaving the apparatus.

The apparatus may optionally be provided with means to promote forced circulation of air inside it and/or with baffle and /or other devices intended to control the duration of the contact between air to be treated and the infrared and ultra-violet radiations.

In this manner the apparatus acts by killing germs and/or by modifying substances suspended in the air to be breathed, eliminating a large portion and/or substantially attenuating the factors that cause some allergies and/or other illnesses affecting human health and allowing the body to better resist the damaging action of attacking agents. In addition to these effects, the continued used of the apparatus promotes the strenthening of the body defenses and therefore acts as a real health promoting and purifying agent for the home atmosphere.

The use of an infra-red lamp additional to an ultraviolet lamp, as has been know in similar devices, has the advantages of better killing of bacteria or other germs; and the infra-red lamp causes convection currents which carry the air being treated.

Apparatus of the invention of appropriate size can also be used to treat air in areas larger than the rooms in a home, such as offices, industrial installations or hospitals, to prevent possible contamination.

The invention is now illustrated by reference to the drawing, wherein: Fig. 1 is a schematic vertical sectional view of one embodiment of the apparatus of the invention; Fig. 2 is a schematic vertical sectional view of another embodiment of the apparatus; Fig. 3 is a vertical section of a third embodiment; Fig. 4 is a section through further embodiments of the apparatus, incorporated in a lighting fixture.

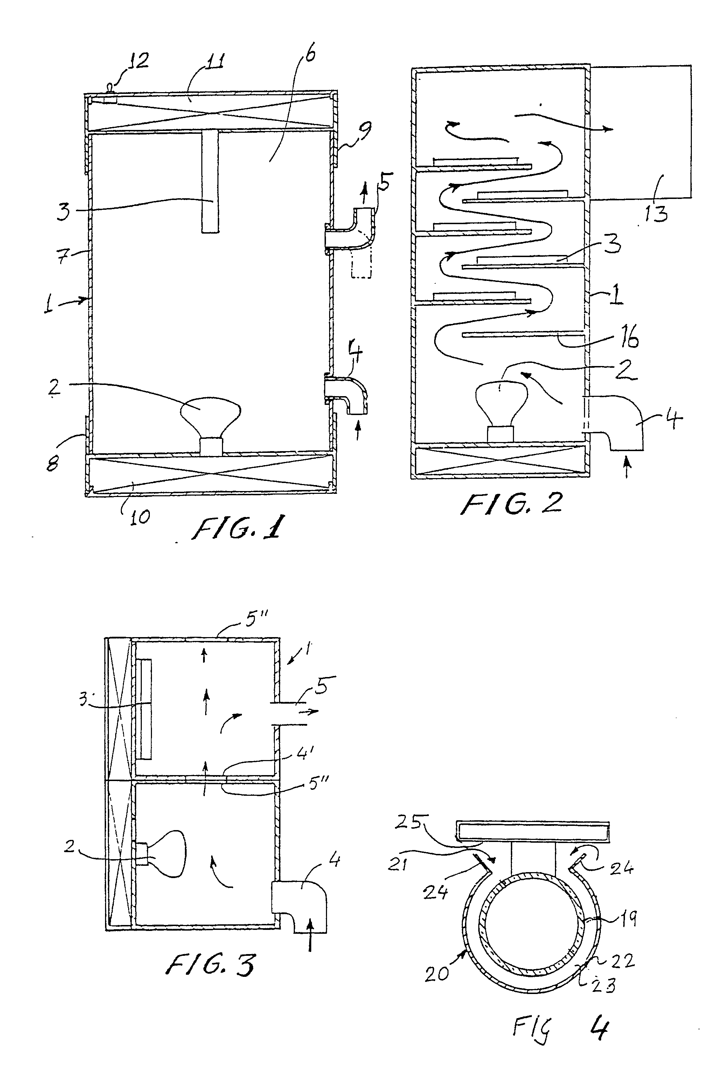

In Figs. 1 to 3 the apparatus essentially comprises a vertical housing (1) made of material that prevents the propagation of infra-red and ultra-violet and other radiation, containing infra-red lamps in its lower part (2) and ultra-violet lamps (UVA-UVB-UVC) in its upper part (3), an entry aperture (4) being provided in the housing wall at the level of the infra-red lamps. Through this aperture, air to be treated will be introduced by convection. An air outlet (5) is provided at a level above the entry; this outlet may be located at an intermediate level in the housing wall, so as to define an air accumulation chamber (6) inside the housing and above said air outlet (5); in this chamber air will be accumulated for a longer time undergoing the action of the lamps, part of this air to be mixed, by means of the Venturi effect, with the flow of treated air that does not accumulate and thus controlling the condition of the air leaving the apparatus.

In Fig. 1, the housing (1) is formed by an intermediate tubular body (7) of any desired cross section, receiving respective lower (8) and upper (9) covers at its ends, to support infra-red lamps (2) and ultra-violet lamps (3) respectively and to define chambers (10) and (11) which house the electrical components that activate the lamps. An On/Off switch (12) may also be attached to the housing (1) or a cover.

In order to regulate the exposure time of the air to be treated by infra-red and ultra-violet radiation, in addition to the accumulation chamber (6) other means may be employed such as making the entry (4) and outlet (5) with permanent diameters that are different in different apparatuses in order to determine different flows, or else making adjustable entry and outlet openings and/or locating them at different either permanent or adjustable distances within the housing body.

Another manner of regulating the exposure time of air to be treated consists in installing lamps of different power rating which as well as acting on the air with different intensities, make it accumulate at a greater or lesser speed between the entry and the outlet, thus regulating the desired levels of action on the air to be treated.

Another way to affect the air circulation rate inside the apparatus and consequently the period of exposure of the air to the infra-red and ultra-violet radiations is to provide the apparatus with internal adjustable electric resistances which will regulate the inside heat at levels corresponding to the desired circulation speed as well as foster the assisting of killing and/or modification of germs.

For the same purpose a forced air circulation system can be provided, which is formed by ventilators suitably disposed inside the apparatus to draw the air introduced through the inlet (4).

Still another way to regulate the action on air circulating through the apparatus would be the installation of infra-red and ultra-violet lamps in adequate numbers, power ratings and arrangements.

Entry (4) and Outlet (5) are preferably formed by "U" or "L" shaped piping, and/or screens could be provided in front thereof and inside the apparatus, all tending to prevent light, heat, ultra-violet, infra-red and other radiations from escaping.

The insulating material employed in the housing construction may be, e.g, of glass, opaque glass and/or painted glass, glass wool with or without resin, polystyrene, cork, plastic, asbestos, stainless steel, wood, plywood or wood agglomerates, these material being used singly or two or more thereof combined to form the protective wall. Provided adequate protection is obtained and observing the functional aspect, any ornamental design may be used on the outside of the housing, like e.g. cylindrical, prismatic or pyramidal figures. The electrical part of the apparatus is provided by the usual means that are suited to the type of lamps or other devices employed, the electrical components that are part of it being preferably installed outside the housing and the connecting devices to the lamps such as contacts or sockets being sealed so as to prevent radiation leakage.

This apparatus is used preferably in the user's bedroom during his sleeping hours at a distance of one or more meter, as a means to prevent allergic processes, that is, before the illness settles deeply into the body it can also be employed in the treament of existing pathological states or by any other way or in other locations, provided the user is exposed for long periods to the treated air from the apparatus. Thus when the apparatus is connected, its inside will heat up, and by mere convection and/or with the help of ventilators, the room air is drawn into its housing and exposed, during a time period to be regulated by the regulating means described, to the infra-red and ultra-violet radiations from the lamps and later returned to the room adequately treated, that is, the germs and substances suspended in it having been destroyed and/or modified. As the user of the apparatus breathes this air, his resistance to aggressive agents that cause allergies and other diseases affecting mainly the respiratory tract will be increased.

In another embodiment the apparatus as shown in Fig. 2, essentially formed by a vertical housing (1), made of insulating material to prevent propagation of light, heat, infra-red and ultra-violet or other radiation, and housing one or more infra-red lamps (2) in its bottom part and one or more ultra-violet lamps in its upper part (3), and having an entry (4) for the air to be treated located at the lower lamp level and an outlet (5) for treated air located in the upper part, a forced ventilation system can be provided preferably an airconditioner,(13) which can also be attached to the apparatus of Fig. 1.

The number of infra-red and ultra-violet lamps can be varied to give performance capacity adequate to the conditions of the enviroment to be protected and/ or to the air-conditioner to be connected.

The apparatus can include means to regulate the time during which the air is exposed to the action of the lamps. These means may be a baffle (14) arranged along the housing and formed by a plurality of glass plates (15) or of any other material allowing the propagation of the radiation emitted by the lamps, parallel to one another, regularly spaced and orthogonally projecting from one of the housing walls and intercalated between similar plates (16) projecting from the opposite wall. The air circulating on a zig-zag path among the plates flows for a longer time in contact with the ultra-violet and infra-red radiations from the lamps.

In the embodiment of Fig, 3, the apparatus may contain only either infra-red or ultra-violet lamps connected in series to obtain environmental protection effects.

Thus, in this type of apparatus we have a housing (1) containing one infra-red lamp. (2) or one ultra-violet lamp (3) with an inlet aperture (4) for the air to be treated, and an outlet for the treated air (5). Several such apparatuses may be connected in series so that the outlet (5$''$) of one of them is coupled to the entry (4$'$) of the next adjacent one.

In any form of construction of the apparatus, filters may be provided in the entrance and outlet ducts.

Of course, the second and third embodiments of the apparatus have their housing provided with a lid for access to the inside, and appropriate compartments to house the electrical components that activate the lamps. The housing is made of insulating material that is capable of retaining ultra-violet and infra-red radiations and the air inlet and outlet are made so as not to allow leakage of these radiations, and may receive any suitable exterior finish.

The infra-red lamps (2) can be ordinary tungsten filament light bulbs.

The invention also embraces the use of the apparatus for purifying air.

## Claims

1. Apparatus for purifying air by causing a stream of air to be subjected to ultraviolet radiation, which comprises a vertically elongated housing (1) made of material which is opaque to infrared, ultra-violet, heat and other radiation, containing infra-red lamps in its lower part (2) and ultra-violet lamps (UVA-UVB-UVC) in its upper part (3), with an inlet aperture (4) provided in the housing wall at a lower level of the housing, through which aperture, when the lamps are illuminated, air to be treated is introduced by convection, and above this inlet is provided an outlet for treated air (5), which may be located at an intermediate level of the housing so as to define an air accumulation chamber (6) located above said air outlet, whereby in this chamber air is confined and exposed to the action of the lamps, part of this air being mixed with the treated air leaving the apparatus.

2. Apparatus as claimed in Claim 1, wherein the housing (1) is formed by an intermediate tubular body (7) having at its respective ends a lower cover (8) and an upper cover (9) that respectively support the infra-red lamps (2) and ultra-violet lamps (3) and the covers also define external chambers (10) and (11) housing electrical components that activate the lamps.

3. Apparatus as claimed in Claim 1 or 2, which includes means to prolong the time of exposure of the air to the ultraviolet lamps, these means being constituted by permanent different diameter inlet (4) and outlet (5) apertures,or the provision of an inlet (4) and outlet (5) with adjustable diameters, and/or the arrangement thereof at different fixed distances from each other or at adjustable distances from each other.

4. Apparatus as claimed in Claim 1, 2 or 3, wherein means to control the duration of exposure of air to the ultraviolet lamps consists of the lamps (2), (3) being of different power ratings and/or present in different amounts and/or the inclusion of electrical resistances in series with the lamps in appropriate amounts and/or power ratings, in order to control heat inside the apparatus and consequently the air flow rate.

5. Apparatus as claimed in Claim 1, 2 or 3, wherein the means to control the duration of exposure of air to the ultraviolet lamps consists of a forced air circulation device formed by ventilators inside the housing (1).

6. Apparatus as claimed in any preceding claim, wherein the inlet (4) and outlet (5) are provided with means to prevent radiation leakage, consisting in the inlet and outlet ducts haveing a "U" or "L" shape, and/or that a screen is provided in front of their aperture opening into the inside of the housing(1).

7. Apparatus as claimed in any preceding claim, wherein to prolong the duration of exposure of air to the action of the ultra-violet (3) lamps, the housing contains a baffle (14) formed by a plurality of plates (15) permitting the propagation of radiation, and forming a zig-zag path for the air between said plates.

8. Apparatus as claimed in any preceding claim, in which an individual housing is provided only with either infra-red lamps (2) or ultra-violet lamps (3), and a plurality of these housings form a set, arranged in series so that the outlet of one housing is connected to the inlet of the next adjacent housing.

## Revendications

1. Appareil pour purifier l'air en soumettant un courant d'air à un rayonnement ultraviolet, qui comprend une enceinte (1) verticalement allongée constituée d'une matière qui est opaque vis-à-vis des rayonnements infrarouges, ultraviolets, thermiques ou autres, qui contient des lampes à infrarouge dans sa partie inférieure (2) et des lampes à ultraviolet (UVA-UVB-UVC) dans sa partie supérieure (3), avec une ouverture d'entrée (4) prévue dans la paroi de l'enceinte à un niveau inférieur de l'enceinte, ouverture à travers laquelle, lorsque les lampes sont allumées, l'air à traiter est introduit par convection, et où, au-dessus de cette entrée, il est prévu une sortie pour l'air traité (5), qui peut être située à un niveau intermédiaire de l'enceinte de façon à définir une chambre d'accumulation d'air (6) située au-dessus de ladite sortie, avec le résultat que dans cette chambre l'air est confiné et exposé à l'action des lampes, une partie de cet air étant mélangée à l'air traité quittant l'appareil.

2. Appareil suivant la revendication 1, dans lequel l'enceinte (1) est formée par un corps tubulaire intermédiaire (7) présentant à ses extrémités respectives un couvercle inférieur (8) et un couvercle supérieur (9) qui supportent respectivement les lampes à infrarouge (2) et les lampes à ultraviolet (3) et où les couvercles définissent aussi des chambres externes (10) et (11) hébergeant des composants électriques qui activent les lampes.

3. Appareil suivant la revendication 1 ou 2, qui comprend des moyens pour prolonger le temps d'exposition de l'air aux lampes à ultraviolet, ces moyens étant constitués par des ouvertures permanentes d'entrée (4) et de sortie (5) de diamètre différent, ou par la présence d'une entrée (4) et d'une sortie (5) de diamètre réglable, et/ou par l'agencement de celles-ci à différentes distances fixes l'une de l'autre ou à des distances réglables l'une de l'autre.

4. Appareil suivant l'une des revendications 1, 2 ou 3, dans lequel les moyens de réglage de la durée d'exposition de l'air aux lampes à ultraviolet consistent en ce que les lampes (2), (3) ont des puissances différentes et/ou sont en nombres différents et/ou en ce que l'on prévoit d'insérer des résistances électriques en série avec les lampes en quantités et en puissances appropriées, afin de régler la chaleur à l'intérieur de l'appareil et par conséquent le débit d'air.

5. Appareil suivant l'une des revendications 1, 2 ou 3, dans lequel les moyens de réglage de' la durée d'exposition de l'air aux lampes à ultraviolet se composent d'un dispositif de circulation forcée de l'air constitué de ventilateurs montés à l'intérieur de l'enceinte (1).

6. Appareil suivant l'une ou l'autre des revendications précédentes, dans lequel l'entrée (4) et la sortie (5) sont pourvues de moyens pour empêcher les fuites de rayonnements, consistant en ce que les conduits d'entrée et de sortie ont une forme en "U" ou en "L", et/ou en ce qu'il est prévu un écran en face de leur ouverture à l'intérieur de l'enceinte (1).

7. Appareil suivant l'une ou l'autre des revendications précédentes, dans lequel, pour prolonger la durée d'exposition de l'air à l'action des lampes à ultraviolet (3), l'enceinte contient un déflecteur (14) formé par une pluralité de plaques (15) permettant la propagation du rayonnement et formant un chemin en zig-zag pour l'air entre lesdites plaques.

8. Appareil suivant l'une ou l'autre des revendications précédentes, dans lequel une enceinte individuelle est pourvue uniquement de lampes soit à infrarouge (2) soit à ultraviolet (3), et où une pluralité de ces enceintes forment un ensemble arrangé en série de sorte que la sortie d'une enceinte soit raccordée à l'entrée de l'enceinte adjacente suivante.

## Ansprüche

1. Vorrichtung zur Luftreinigung, indem man auf einen Luftstrom ultraviolette Strahlung einwirken läßt, die ein in senkrechter Richtung längliches Gehäuse (1) umfaßt, das aus einem für Infrarotstrahlung, ultraviolette Strahlung, Wärmestrahlung und sonstige Strahlung undurchlässigen Material besteht und das in seinem unteren Teil (2) Infrarotlampen und in seinem oberen Teil (3) ultraviolette Lampen (UVA-UVB-UVC) aufweist, wobei in der Gehäusewand weiter unten am Gehäuse eine Einlaßöffnung (4) vorgesehen ist, durch die bei brennenden Lampen zu behandelnde Luft durch Konvektion eingeführt wird, und über diesem Einlaß ein Auslaß für behandelte Luft (5) vorgesehen ist, der sich auf mittlerer Gehäusehöhe befinden kann, um eine sich über jenem Luftauslaß befindende Luftsammelkammer (6) zu begrenzen, wodurch in dieser Kammer Luft eingeschlossen und der Wirkung der Lampen ausgesetzt wird, wobei ein Teil dieser Luft mit der die Vorrichtung verlassenden, behandelten Luft vermischt wird.

2. Vorrichtung nach Anspruch 1, bei der das Gehäuse (1) von einem rohrförmigen Zwischenkörper (7) gebildet wird, der an seinen jeweiligen Enden eine untere Abdeckung (8) und eine obere Abdeckung (9) aufweist, die jeweils die Infrarotlampen (2) und die ultravioletten Lampen (3) stützen und außerdem äußere Kammern (10) und (11) begrenzen, in denen elektrische Bauteile untergebracht sind, die die Lampen betätigen.

3. Vorrichtung nach Anspruch 1 oder 2, die Mittel zur Verlängerung der Einwirkungszeit der ultravioletten Lampen auf die Luft einschließt, wobei diese Mittel aus Einlaß- (4) und Auslaßöffnungen (5) mit permanentem, unterschiedlichem Durchmesser bestehen oder ein Einlaß (4) und Auslaß (5) mit einstellbaren Durchmessern vorgesehen sind und/oder diese in unterschiedlichen, festen Abständen oder in einstellbaren Abständen zueinander angeordnet sind.

4. Vorrichtung nach Anspruch 1, 2 oder 3, bei der das Mittel zur Steuerung der Einwirkungsdauer der ultravioletten Lampen auf die Luft, aus den Lampen (2), (3) besteht, die unterschiedliche Nennleistungen haben und/oder in unterschiedlicher Anzahl vorliegen und/oder elektrische Widerstände in angemessener Zahl und/oder Nennleistungen mit den Lampen in Reihe geschaltet werden, um die Wärme in der Vorrichtung und folglich die Luftströmungsgeschwindigkeit zu steuern.

5. Vorrichtung nach Anspruch 1, 2 oder 3, bei der das Mittel zur Steuerung der Einwirkungsdauer der ultravioletten Lampen auf die Luft, aus einer durch Ventilatoren im Gehäuse (1) gebildeten Luftumwälzeinrichtung besteht.

6. Vorrichtung nach einem der vorhergehenden Ansprüche, bei der der Einlaß (4) und Auslaß (5) mit einem Mittel zur Verhinderung von Strahlungsleckage versehen sind, das darin besteht, daß die Ein- und Auslaßkanäle Uoder L-förmig sind und/oder daß vor ihrer in das Innere des Gehäuses (1) mündenden Öffnung eine Blende vorgesehen ist.

7. Vorrichtung nach einem der vorhergehenden Ansprüche, bei der zur Verlängerung der Einwirkungsdauer der ultravioletten (3) Lampen auf die Luft, das Gehäuse eine aus einer Mehrzahl von die Strahlungsausbreitung gestattenden Platten (15) gebildete Sperre (14) enthält, durch die die Luft auf einem Zickzackkurs zwischen jenen Platten geführt wird.

8. Vorrichtung nach einem der vorhergehenden Ansprüche, bei der ein einzelnes Gehäuse nur entweder mit Infrarotlampen (2) oder mit ultravioletten Lampen (3) versehen ist, und eine Mehrzahl dieser Gehäuse einen in Reihe geschalteten Satz bilden, so daß der Auslaß eines Gehäuses mit dem Einlaß des nächsten, benachbarten Gehäuses verbunden ist.

FIG. 1

FIG. 2

FIG. 3

FIG 4